# EUROPEAN PATENT SPECIFICATION

(11) **EP 0 975 296 B1**
(45) Date of publication and mention of the grant of the patent: **22.12.2004**
(21) Application number: 97947291.7
(22) Date of filing: 28.10.1997
(51) Int. Cl.: A61F 13/15

(54) **DISPOSABLE PULL-ON GARMENT HAVING IMPROVED TEAR OPEN HANDLING AFTER SOIL**
WEGWERFÜBERZIEHKLEIDUNGSSTÜCK MIT VERBESSERTER AUFREISSHANDHABUNG NACH BESCHMUTZUNG
VETEMENT A ENFILER JETABLE A OUVERTURE AMELIOREE PAR DECHIRURE LORSQUE LEDIT VETEMENT A ETE SOUILLE

(30) Priority: 30.10.1996 EP 96117394; 30.05.1997 WO PCT/US97/00097
(43) Date of publication of application: 02.02.2000
(73) Proprietor: THE PROCTER & GAMBLE COMPANY, Cincinnati, Ohio 45202 (US)
(72) Inventor: SCHMITZ, Christoph, Johann, D-53881 Euskirchen (DE); CHAN, John, Geoffrey, Guangzhou 510010 (CN)
(74) Representative: Canonici, Jean-Jacques
(86) International application number: PCT/US1997/019561
(87) International publication number: WO 1998/018421

(56) References cited:
- EP-A- 0 483 692
- EP-A- 0 657 153
- WO-A-89/07897
- WO-A-95/34266
- WO-A-96/31178
- US-A- 4 610 680
- US-A- 4 619 649
- US-A- 4 909 804
- US-A- 5 074 854
- US-A- 5 451 219
- US-A- 5 496 298
- US-A- 5 516 567
- US-A- 5 662 638

## Description

### FIELD

The present invention relates to disposable pull-on garments. Examples of such disposable pull-on garments include disposable underwear, pull-on diapers, training pants, and disposable panties for menstrual use. The present invention more particularly relates to unitary disposable absorbent pull-on garments such as pull-on diapers, training pants, incontinent pull-on briefs, and the like, which provide improved tear open handling after soil.

### BACKGROUND

Infants and other incontinent individuals wear disposable absorbent articles such as diapers to receive and contain urine and other body exudates. Absorbent garments having fixed sides (e.g., training pants or pull-on diapers) have become popular for use on children able to walk and often who are toilet training. In order to contain body exudates as well as to fit a wide variety of body shapes and sizes, these pants must fit snugly about the waist and legs of the wearer without drooping, sagging or sliding down from its position on the torso as well as fitting larger wearers without causing irritation to the skin due to the product being too tight. Thus, the pant must have elastic extensibility in the waist and legs with the elastic features providing a high degree of stretch.

Many training pants and pull-on diapers use conventional elastic elements secured in an elastically contractible condition in the waist and leg openings. Typically, in order to insure full elastic fit about the leg and the waist such as is provided with durable undergarments, the leg openings and waist opening are encircled with elasticized bands of rubber or other materials positioned along the curve of the opening. These pants are typically characterized as "balloon style" pants because of the contraction caused by the elasticized bands in specific zones of the product while the remaining material tends to blouse. Examples of such training pants are disclosed in U.S. Patent No. 5,171,239 to Igaue, et al. on December 15, 1992 and U.S. Patent No. 4,610,681 to Strohbeen, et al. on September 9, 1986.

These training pants typically have side panels whose edges are seamed together to form two leg openings and a waist opening. In one typical seaming manner, the body-facing sides of the seam panels are seamed together at their edges. Examples of such seaming manner are disclosed in the above referenced U.S. Patent Nos. 5,171,239 and 4,610,681. In another typical seaming manner, the body-facing side of one seam panel and the outer-facing side of the other seam panel are seamed together at their edges. An example of such seaming manner is disclosed in U.S. Patent No. 5,569,234 to Buell et al. on October 29, 1996. The latter seaming manner generally provides a stronger seam structure than the former seaming manner. In particular, to secure a better fit of disposable pull-on garments, the seams are required to withstand large forces during application and use. Otherwise, such forces and stresses may cause the side panels to break away or tear open.

While such stronger seam structures are highly desirable, it is also further desired that the bonded or seamed side panels can be easily torn open by a user's fingers after soil. However, because of the strength of the seam, the overlapped seam structure tends to be difficult to tear open after the pull-on garment has been soiled.

EP-A-0 483 692 discloses a pull on type garment with seams and tabs extending outwardly from the seams. WO 89/07897 discloses a pair of part-type rappies provided with a tear strip allowing the briefs to be opened at one of their sides. EP-A-0 657 153 discloses a part type diapat having seams formed by welded zones which are defined by patterns of V-shape.

Based on the foregoing, there is a need for a disposable pull-on garment having side seams that are strongly enough to withstand large forces during application and use, while not excessively interfering with the removal of the garment after soil. None of the existing art provides all of the advantages and benefits of the present invention.

### SUMMARY

The present invention is directed to a disposable pull-on garment. In one aspect of the present invention, the disposable pull-on garment comprising an outer surface, an inner surface, a front portion, a rear portion, a crotch portion, each of the front portion and the rear portion having side panels with side edges, and overlapping side seams which join together the side panels of the front portion and the rear portion to form leg openings and a waist area, characterized in that at least one side flap is disposed outboard of each of the side edges of the side panels forming the outermost portion of the overlapping side seams.

In another aspect of the present invention, the disposable pull-on garment comprises a chassis having a front region, a back region and a crotch region between the front region and the back region. The chassis comprises a liquid pervious topsheet, a liquid impervious backsheet associated with the topsheet, and an absorbent core disposed between the topsheet and the backsheet. The disposable pull-on garment of the present invention further comprises front side panels extending laterally outwardly from each side of the chassis in the front region; back side panels extending laterally outwardly from each side of the chassis in the back region; seam panels each extending laterally outwardly from each of the front and back side panels; a tear open tab extending laterally outwardly from at least one of the seam panels; and seams each joining the corresponding seam panels in an overlap manner to make an overlapped seam structure, thereby forming two leg openings and a waist opening.

The present invention is further directed to a method for making a disposable pull-on garment. In yet another aspect of the present invention, the method comprising the steps of: cutting the side flap from the side edges of the side panels; and joining the side panels of the front portion and the rear portion to form overlapping side seams.

These and other features, aspects, and advantages of the present invention will become evident to those skilled in the art from a reading of the present disclosure.

### BRIEF DESCRIPTION OF THE DRAWINGS

While the specification concludes with claims particularly pointing out and distinctly claiming the invention, it is believed that the invention will be better understood from the following description of preferred embodiments which is taken in conjunction with the accompanying drawings and which like designations are used to designate substantially identical elements, and in which:
Fig. 1 is a perspective view of a preferred embodiment of the disposable pull-on garment according to one aspect of the present invention in a typical in use configuration;
Fig. 2 is a simplified plan view of the embodiment shown in Fig. 1 in its flat uncontracted condition showing the various panels or zones of the garment;
Fig. 3 is a plan view of the embodiment shown in Fig. 1 in its flat uncontracted condition showing the outer surface and having portions cut away to reveal underlying structure;
Fig. 4 is a fragmentary view of the pull-on garment shown in Fig. 3 taken along section line 4-4 of Fig. 3;
Fig. 5 is a plan view of the pull-on garment shown in Fig. 1 in its flat uncontracted condition showing the inner surface and having portions cut away to reveal underlying structure;
Fig. 6 is a plan view of the tear open tab and seam panel of a preferred embodiment of the present invention;
Fig. 7A is a fragmentary cross-sectional view of the seam panels taken along line 7-7 of Fig. 6, showing forces generated during wear;
Fig. 7B is a fragmentary cross-sectional view of the seam panels shown in Fig. 7A when a pull-on diaper is torn open;
Fig. 8 is a perspective view of a preferred embodiment of a pull-on diaper when it is folded after soil;
Fig. 9 is a perspective view of a preferred embodiment when the pull-on diaper shown in Fig. 8 is secured in a configuration for disposal;
Fig. 10 is a perspective view of another preferred embodiment when the pull-on diaper shown in Fig. 8 is secured in another configuration for disposal; and
Fig. 11 is a perspective view of a preferred embodiment of the disposable pull-on garment according to another aspect of the present invention.

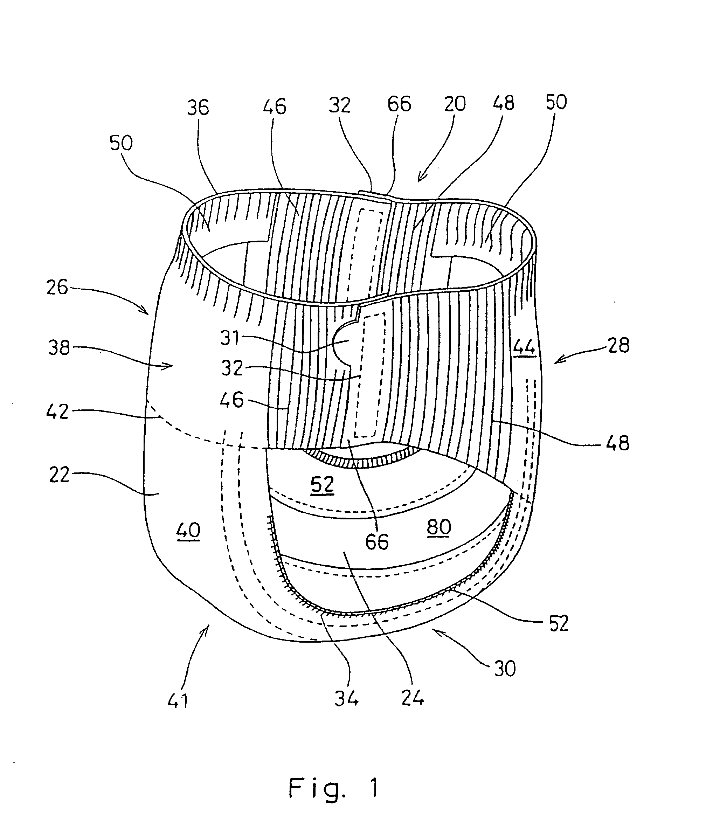

As used herein, the term "pull-on garment" refers to articles of wear which have a defined waist opening and a pair of leg openings and which are pulled onto the body of the wearer by inserting the legs into the leg openings and pulling the article up over the waist. The term "disposable" is used herein to describe garments which are not intended to be laundered or otherwise restored or reused as a garment (i.e., they are intended to be discarded after a single use and, preferably, to be recycled, composted or otherwise disposed of in an environmentally compatible manner). A "unitary" pull-on garment refers to pull-on garments which are formed of separate parts united together to form a coordinated entity, but the side panels are not separate elements joined to a separate chassis in that the side panels are formed by at least one layer which also forms the central panel or chassis of the garment (i.e., the garment does not require separately manipulative panels such as a separate chassis and separate side panels). The pull-on garment is also preferably "absorbent" to absorb and contain the various exudates discharged from the body. A preferred embodiment of the pull-on garment of the present invention is the unitary disposable absorbent pull-on garment, pull-on diaper 20, shown in Fig. 1. As used herein, the term "pull-on diaper" refers to pull-on garments generally worn by infants and other incontinent individuals to absorb and contain urine and feces. It should be understood, however, that the present invention is also applicable to other pull-on garments such as training pants, incontinent briefs, feminine hygiene garments or panties, and the like. As used herein, the term "panel" is used herein to denote an area or element of the pull-on garment. (While a panel is typically a distinct area or element, a panel may coincide (functionally correspond) somewhat with an adjacent panel.) As used herein, the term "joined" or "joining" encompasses configurations whereby an element is directly secured to the other element by affixing the element directly to the other element, and configurations whereby the element is indirectly secured to the other element by affixing the element to intermediate member(s) which in turn are affixed to the other element. The term "tear open tab" refers to a short projecting or protruding device, which can be grasped and can be used to tear open the disposable pull-on diaper.

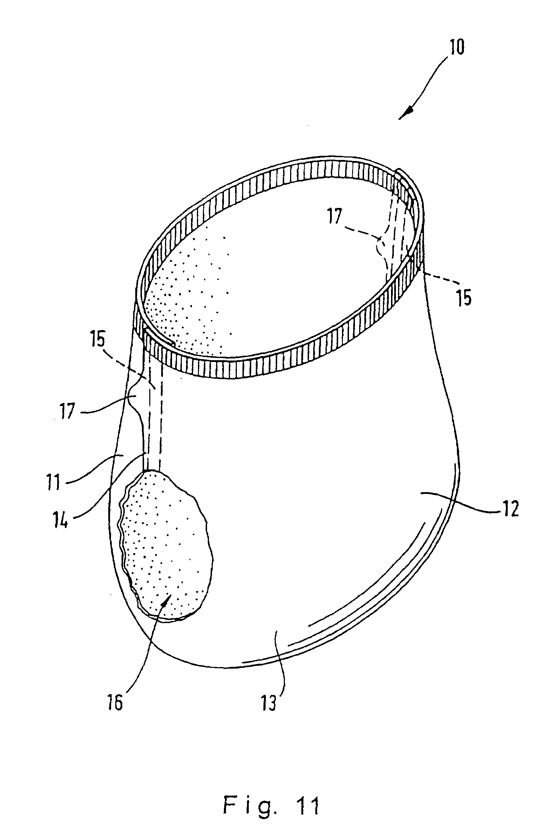

Fig. 11 is a perspective view of the disposable pull-on diaper 10 according to one aspect of the present invention. The pull-on diaper 10 has an outer surface, an inner surface, a front portion 11, a rear portion 12, a crotch portion 13, each of said front portion 11 and said rear portion 12 having side panels with side edges 14, overlapping side seams 15 which join together the side panels of the front portion 11 and the rear portion 12 to form leg openings 16 and a waist area, and at least one side flap (or tear open tab) 17 is disposed outboard of each of the side edges 14 of the side panels forming the outermost portions of the overlapping side seams 15. Preferably, the side flap 17 is an integral part of the disposable pull-on diaper. The pull-on diaper 10 preferably comprises a chassis layer; an elastically extensible stretch laminate positioned in each side panel of the front portion 11, front stretch laminates; an elastically extensible stretch laminate positioned in each side panel of the rear portion 12, rear stretch laminates; and at least one elasticised waistband positioned in both the front portion 11 and the rear portion 12. The pull-on diaper 10 comprise leg openings 16 which additionally comprise elastic leg features to improve fit at the legs in the crotch portion 13. The pull-on diaper 10 can be furnished with apertures or vents (not shown) in at least the side panels of the pull-on diaper 10 to provide breathability and ventilation.

The pull-on diaper 10 has a crotch portion 13 comprising a main panel and a pair of leg flap panels. The absorbent core is generally positioned within the main panel of the crotch portion 13 since bodily exudates are typically discharged in this area. A leg flap panel extends generally laterally outwardly from and along each side edge of the main panel. Each leg flap panel generally forms at least a portion of the elastic leg feature. The outer surface of the pull-on diaper 10 comprises that portion which is positioned away from the body of the wearer during use. The inner surface of the diaper 10 is opposed to the outer surface and comprises that portion of the diaper which is positioned adjacent to the body of the wearer during use.

Elastically extensible stretch laminates (front stretch laminates and rear stretch laminates) are formed in each side panel of both the front portion 11 and the rear portion 12. Each stretch laminate is mechanically stretched or drawn to allow the stretch laminate to be elastically extensible in at least the lateral direction. (The lateral direction (x direction or width) is defined as the direction parallel to the lateral centreline of the pull-on diaper 10. The side panels are preferably an extension of the chassis layer and other elements such as the topsheet, or any other combination of these elements. In the overlapping side seams 15, the stretch laminate is preferably activated by mechanical stretching to provide additional extensibility in this region. The overlapping side seams 15 may also not be activated by mechanical stretching.

In order to provide the necessary absorbency to contain bodily discharges, the pull-on diaper 10 comprises a liquid pervious topsheet and an absorbent core positioned between the topsheet and the chassis layer. The topsheet is positioned adjacent to the body surface of the absorbent core and is preferably joined to the absorbent core and the chassis layer by attachment means such as those well known in the art. In a preferred embodiment, the topsheet and the chassis layer are indirectly joined together by directly joining them to the absorbent core or the elastic panel members or other elements of the pull-on diaper. The topsheet preferably comprises three distinct layers joined together. A liquid pervious primary layer is positioned over the absorbent core to rapidly absorb liquids into the product. Barrier layers are joined to the primary layer and are preferably drawable, more preferably hydrophobic, to allow the side panels to be mechanically stretched without ripping or tearing while providing barrier cuffs along the sides of the pull-on diaper 10. The elastic leg features preferably comprise a gasketing cuff and a barrier cuff. The gasketing cuff is preferably formed by one or more elastic leg members operatively joined to the chassis layer, the barrier layer, or both, preferably between the chassis layer and the flap portion of the barrier layer in the leg flap panel of the crotch portion 13. The barrier cuff is preferably formed by a flap (the stand-up portion of the barrier layer, closing means for securing the longitudinal ends of the stand-up portion to the primary layer, and an elastic spacing member operatively joined to the stand-up portion.

The primary layer is preferably compliant, soft feeling, and non-irritating to the skin of the wearer. The primary layer is liquid pervious permitting liquids (e.g., urine) to readily penetrate through its thickness. A suitable primary layer may be manufactured from a wide range of materials, such as porous foams; reticulated foams; apertured plastic films or three dimensionally expanded formed films; or woven or nonwoven webs of natural fibers, synthetic fibers, or a combination of natural and synthetic fibers. Preferably, the primary layer is manufactured by Amoco. The primary layer is preferably non-coterminous with the chassis layer so that liquid will not wick along and through the primary layer to the edges of the pull-on diaper 10, so that liquids will not wick underneath and beyond the stand-up barrier cuffs formed by the barrier layers, and so that more drawable materials may be positioned in the side panels to produce stronger stretch laminates. The primary layer preferably overlays a major portion of the body surface of the absorbent core, more preferably all of the body surface area of the absorbent core in at least the crotch portion 13, so that exudates that are discharged into the pull-on diaper 10 penetrate through the primary layer where they are absorbed by the absorbent core. The primary layer extends laterally outwardly toward the side edges of the absorbent core, preferably beyond the side edges of the absorbent core in at least the crotch portion 13. The primary layer, however, terminates inwardly of the leg edges of the crotch portion 13. In the most preferred configurations, the primary layer terminates adjacent the proximal edge of the barrier layer (i.e., the terminating edge of the primary layer is positioned adjacent to the proximal edge) or the terminating edge is positioned remotely from and inboard of the proximal edge. "Adjacent" is used in this context to mean that the primary layer terminates at the proximal edge plus or minus small areas of the primary layer material that may extend inside or beyond the proximal edge due to machine tolerances during manufacture or variations in the area of the primary layer when it is manufactured. In the preferred embodiment of the topsheet, the barrier layers form the elastic leg features (preferably, a gasketing cuff and/or a barrier cuff) and, preferably, a portion of the stretch laminates.

The chassis layer preferably comprises a continuous sheet or web which defines the front portion 11, the rear portion 12, and the crotch portion 13. Thus, the chassis layer is the primary stratum or layer of the pull-on diaper 10. (As used herein, the term "layer" does not necessarily limit the element to a single strata of material in that a layer may actually comprise laminates or combinations of sheets or webs of the requisite type of materials.) The chassis layer has an inner surface and an outer surface. The inner surface and outer surface of the chassis layer correspond in their orientation to the inner surface and the outer surface of the pull-on diaper 10.

In preferred embodiments, the chassis layer generally determines the overall shape of the pull-on diaper 10. The chassis layer acts as the main structural layer of the pull-on diaper 10 to which other features may be added or joined. The chassis layer is thus positioned in all or most of the surface area of the pull-on diaper 10, although in certain embodiments certain portions of the chassis layer may be apertured, cut-out or removed ("windowed") to enhance stretchability and/or breathability of the pull-on diaper 10 or features of the pull-on diaper 10 in that area. The chassis layer thus may comprise a continuous sheet or web that does not have "joints" or seams such that forces are distributively transmitted through the entire layer or the chassis layer may comprise a continuous sheet or web that does have "joints" with the elasticised leg cuffs 12. As previously discussed herein, the continuous sheet or web of the chassis layer can comprise a single web of material or a laminate of several continuous webs or layers of different materials. The chassis layer may form the outer surface, the inner surface, or portions of either or both, or may be entirely positioned in the interior of the pull-on diaper 10. The chassis layer preferably forms the outer surface of the pull-on diaper 10 in the crotch portion 13.

Since at least a portion of the chassis layer is subjected to mechanical stretching in order to provide the stretch laminates in the side panels, it is preferably elongatable, more preferably drawable (but not necessarily elastomeric), so that the chassis layer will, upon mechanical stretching, be at least to a degree permanently elongated such that it will not fully return to its original undistorted configuration. The chassis layer may thus comprise any of the materials known for use in absorbent articles such as woven or nonwoven webs; polymeric films such as thermoplastic films of polyethylene, polypropylene, or blends thereof; laminates of such materials; or composite materials. In preferred embodiments, the chassis layer can be subjected to mechanical stretching with minimal or no rupturing or tearing. Therefore, the chassis layer is preferably a polymeric film.

Due to the fact that the chassis layer is preferably a polymeric film, it is also generally impervious to liquids (e.g., urine) so that it may also serve as the component which prevents bodily discharges absorbed and contained in the absorbent core from wetting garments which contact the pull-on diaper 10 such as bed sheets and undergarments (i.e., it acts as the traditional diaper backsheet). If the chassis layer is not liquid impervious, typically an additional layer such as a traditional backsheet should be used behind the absorbent core. The chassis layer may also be breathable (pervious to air or water vapour) if desired. The chassis layer can alternatively comprise breathable materials that are microporous and that are, typically, lower in strength and elongation. An example of such a film is that manufactured by Exxon Chemical Company under the tradename EXXAIRE. Exemplary films for use as the chassis layer having relatively good drawability but that are not breathable include polymeric films manufactured by Clopay Corporation of Cincinnati, Ohio under the designation Clopay 1401, or films available from Tredegar of Terre Haute, Indiana, under the designation X-8323 or X-9954.

The size of the chassis layer is dictated by the size of the wearer the pull-on diaper 10 is designed to fit. In a preferred embodiment, the chassis layer has a modified hourglass shape to better fit the wearer. In an embodiment designed to fit large toddlers (about 9 kg to about 15.4 kg), the chassis layer is preferably about 483 millimetres long by about 234 millimetres wide in the front region and the back region and about 165 millimetres wide in the crotch region. The side panels are about 41 millimetres wide, the activated portion of the side panels is about 32 millimetres wide, and the overlapping side seams 15 are about 8.5 millimetres wide. (The actual area of overlap of the overlapping side seams 15 is about 11 millimetres in the embodiment shown herein.) The front portion 11 is about 114 millimetres long, the back portion 12 is about 165 millimetres long, and the crotch portion 13 is about 220 millimetres long.

Seams are formed by bonding together the side panels of the front portion 11 to the side panels of the rear portion 12. The bonding of the seams can be by any suitable means well known in the art appropriate for the specific materials employed in the seam panels. Thus, sonic sealing, heat sealing, pressure bonding, adhesive bonding, sewing, autogeneous bonding, and the like may be appropriate techniques. In a preferred embodiment, the side panels of the front portion 11 and the side panels of the rear portion 12 are joined to form overlapping side seams 15. The overlapping side seams 15 are joined by a pattern of heat/pressure or ultrasonic welds. In this particular embodiment, the seams comprises an intermittent pattern of individual bonds grouped in clusters. Potentially strong overlapping side seams may be produced by increasing certain amounts of polymeric material in the side seam panels 15. The amount of polymeric material in the side seams 15 can be increased by using higher basis weight nonwoven materials, thicker plastic films, or by introducing additional layers of materials to the side panels. For example, additional plastic films or nonwoven webs may be joined in the side panels. Alternatively, the layers forming the pull-on diaper 10 may be extended beyond the intended area of seaming and folded back into the side panel to introduce additional strata in the side panels. Examples of these types of seams are discussed in the above-referenced U.S. 5,236,430.

The pull-on diaper 10 comprises a leg area 14 that comprises elasticised leg cuffs 12 for providing improved containment of liquids and other bodily exudates. The elastic leg features provide improved containment of liquids and other body exudates in the crotch portion 13 and about the leg openings 16 in general. Each elastic leg feature may comprise several different embodiments for reducing the leakage of body exudates in the leg flap panels (the elastic leg feature can be and is sometimes also referred to as leg bands, side flaps, barrier cuffs, or elastic cuffs.) US 3,860,003 describes a disposable diaper which provides a contractible leg opening 16 having a side flap and one or more elastic panel members to provide an elasticized leg cuff (gasketing cuff). US 4,909,803 discloses a disposable diaper having "stand-up" elasticized flaps (barrier cuffs) to improve the containment of the leg regions. U.S. 4,695,278 teaches a disposable diaper having dual cuffs including a gasketing cuff and a barrier cuff. US 4,795,454 discloses a disposable diaper having leakage resistant dual cuffs wherein the topsheet stops short of the side edge of the diaper to prevent wicking out to the side of the garment. While each elastic leg feature may be configured so as to be similar to any of the leg bands, side flaps, barrier cuffs or elastic cuffs described above, it is preferred that each elastic leg feature comprise a combination of a gasketing cuff and a barrier cuff. The gasketing cuff and barrier cuffs are preferably formed as shown in US 4,795,454.

The absorbent core is preferably positioned adjacent to the inner surface of the chassis layer and is preferably joined thereto by attachment means such as those well known in the art. For example, the chassis layer may be secured to the absorbent core by a uniform continuous layer of adhesive, a patterned layer of adhesive, or an array of separate lines, spirals, or spots of adhesive. Alternatively, the attachment means may comprise heat bonds, pressure bonds, ultrasonic bonds, dynamic mechanical bonds, or any other suitable attachment means or combinations of these attachment means as are known in the art.

The absorbent core may be any absorbent means which is generally compressible, conformable, non-irritating to the skin of the wearer, and capable of absorbing and retaining liquids such as urine and other certain bodily discharges. The absorbent core may be manufactured in a variety of sizes and shapes (e.g., rectangular, hour-glass, "T"-shaped, asymmetric, etc.) and from a wide variety of liquid absorbent materials commonly used in disposable diapers and other absorbent articles such as comminuted wood pulp which is generally referred to as airfelt. Examples of other suitable absorbent materials include creped cellulose wadding, meltblown polymers including coform, crosslinked cellulosic fibers, tissue including tissue wraps, absorbent foams, absorbent sponges, superabsorbent polymers, absorbent gelling materials, or any equivalent materials or combinations of materials. The configuration and construction of the absorbent core may also be varied (e.g., the absorbent core may have varying caliper zones, hydrophilic gradients, superabsorbent gradients, or lower average density and lower average basis weight acquisition zones; or may comprise one or more layers or structures). The total absorbent capacity of the absorbent core should, however, be compatible with the design loading and the intended use of the pull-on diaper 10. Further, the size and absorbent capacity of the absorbent core may be varied to accommodate wearers ranging from infants through adults.

A preferred embodiment of the absorbent core has an asymmetric, modified hourglass shape and has a body surface toward the body of the wearer (inner surface) and a garment surface opposite the body surface. An exemplary absorbent structure for use as the absorbent core that has achieved wide acceptance and commercial success is described in US 5,360,420. Preferably, the absorbent core will comprise an acquisition/distribution layer of chemically stiffened cellulosic fibers and a storage layer positioned beneath the acquisition/distribution layer comprising a mixture of wood pulp fibers and superabsorbent material such as are disclosed in US 4,610,478.

The pull-on diaper 10 can also preferably provided with vents or apertures to permit the passage of air and water vapour to and from the interior of the pull-on diaper 10. In a preferred embodiment, the apertures are positioned in the side panels. In this configuration, bodily discharges are prevented from leaking out of the areas adjacent to the absorbent core but air and water vapour are allowed to be exchanged in the product to ventilate it so that the product does not become excessively wetted by body perspiration and uncomfortable to wear. Vents may additionally be provided in other panels of the pull-on diaper 10 or on certain of the features of the pull-on diaper 10 such as the waistband. The pull-on diaper 10 would preferably have a plurality of vents within the side panels, the vents being arranged in a defined pattern of large and small apertures. Breathability may alternatively be provided by making the materials of the pull-on diaper 10 out of air or vapour permeable materials such as are known in the art. For example, the chassis layer could comprise a breathable (vapour permeable) but liquid impervious plastic film. The elastic panel members may be open material such as foams, scrims, nonwovens, or breathable elastomeric films to further enhance the breathability of the pull-on diaper 10. In a particularly preferred embodiment, the waistband is breathable to allow water vapour to escape from the front portion 11 and the rear portion 12 of the pull-on diaper 10. Breathability may be provided in the waistband by selecting relative breathable materials for its construction and/or by aperturing or venting the waistband such as is discussed herein with respect to the stretch laminates in the side panels. In another embodiment, the waistband may be hydrophobic, hydrophilic, or a combination hydrophobic/hydrophilic member. A hydrophilic waistband may be used to pull moisture away from the skin of the wearer to keep the skin from becoming hydrated. Alternatively, a hydrophobic waistband may be used to prevent fluid absorbed by the diaper 10 from leaking out through the waist opening. A combination hydrophobic/hydrophilic waistband may be used to prevent fluid absorbed by the diaper 10 from leaking out through the waist opening while also pulling moisture away from the skin of the wearer to keep the skin from becoming hydrated.

The present invention also relates to a method for making a disposable pull-on diaper 10. In relation to Fig. 11, before joining the side panels of the front portion 11 to the side panels of the rear portion 12, the side flap or tear open tab 17 is cut from the side edges 14 of the side panels forming eventually the outermost portions of the overlapping side seams 15. For this embodiment, the tear open tab 17 is cut from the side edge 14 of the side panel of the rear portion 12, i.e., the tear open tab 17 is disposed outboard of the side edges 14 of the side panels. In an alternative embodiment, the side panels of the rear portion 12 may be joined to the side panels of the front portion 11 and for this, the tear open tab 17 is cut from the side edge 14 of the side panel of the front portion 11. The tear open tab 17 has preferably a curved shape for easy handling and is preferably disposed along the side edge 14 of the side panel corresponding to either the waist area, the leg area or the area between the waist area and the leg area. No extra material is needed to create the tear open tab 17. The tear open tab 17 is thus an integral part of the pull-on diaper 10. An alternative to such an integral structure is a tear open tab 17 that can be attached to either the side edge 14 of the side panel of the front portion 11 or the side edge 14 of the side panel of the rear portion 12 of the chassis before assemblage using means well known in the art.

The pull-on diaper 10 can be applied by a caregiver or be self-applied by the wearer. Typically, the waist opening will be expanded to allow the wearer to insert one of their feet into one of the leg openings 16. The other foot is then inserted into the other leg opening 16. The pull-on diaper 10 is then pulled up over the torso of the wearer into its wearing position. The force wall created by the stretch laminates especially assists in self application of the pull-on diaper 10 by forcing the diaper 10 to be pulled up over the buttocks rather than further expanding. The pull-on diaper 10 is then worn and can contain and hold discharged body exudates. The pull-on diaper 10 is removed from the wearer by tearing open the tear open tab 17 disposed outboard of the side edges 14 of the side panels forming the outermost portion of the overlapping side seams 15.

Fig. 1 is a perspective view of the disposable pull-on diaper 20 according to another aspect of the present invention. Referring to Fig. 1, the pull-on diaper 20 comprises a chassis 41 having a front region 26, a back region 28 and a crotch region 30 between the front region 26 and the back region 28. The chassis 41 comprises a liquid pervious topsheet 80, a liquid impervious backsheet 22 associated with the topsheet 80, and an absorbent core 84 (not shown in Fig. 1) disposed between the topsheet 80 and the backsheet 22. The pull-on diaper 20 further comprises front side panels 46 each extending laterally outwardly from the corresponding side of the chassis 41 in the front region 26, and back side panels 48 each extending laterally outwardly from the corresponding side of the chassis 41 in the back region 28. The pull-on diaper 20 further comprises seam panels 66 each extending laterally outwardly from each of the front and back side panels 46 and 48; and tear open tabs 31 each extending laterally outwardly from the seam panels 66. Preferred examples of the pull-on diaper 20 are disclosed in U.S. Patent No. 5,569,234 to Buell et al. on October 29, 1996.

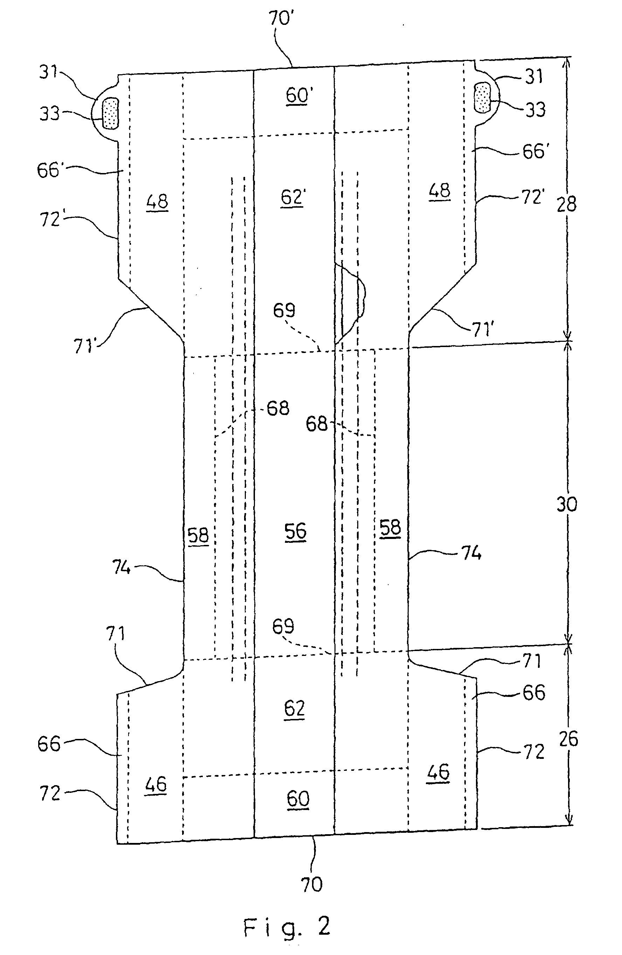

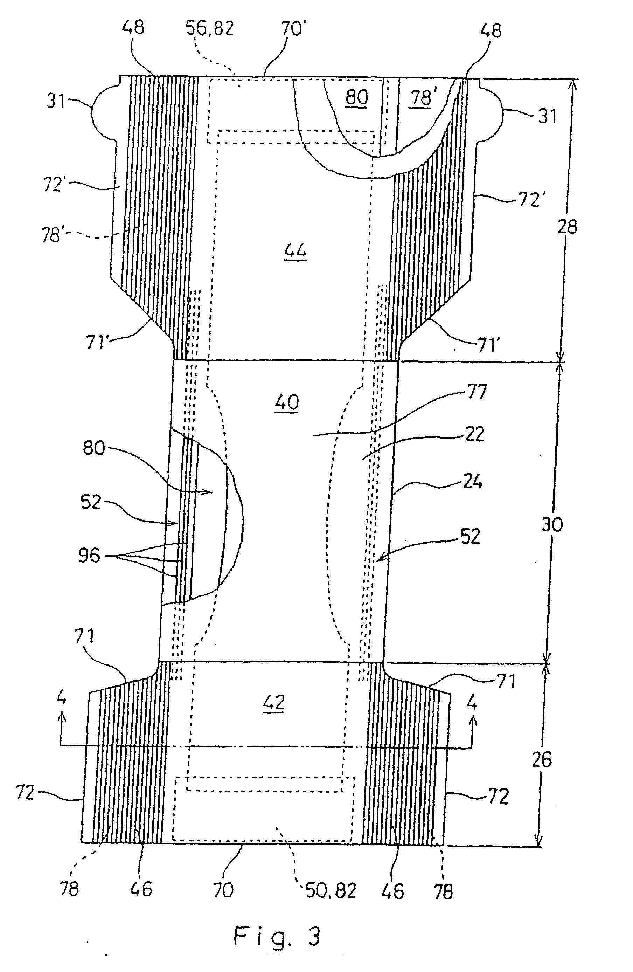

The front and back side panels 46 and 48 of the present invention can be any members which extend laterally outwardly from the corresponding side edges of the chassis 41. In preferred embodiments, each of the front and back side panels 46 and 48 is a projected member which projects laterally outwardly from the chassis 41 (more clearly shown in Figs. 2 and 3). Preferably, the front side panels 46 and back side panels 48 are unitary elements of the pull-on diaper (i.e., they are not separately manipulative elements secured to the pull-on diaper, but rather are formed from and are extensions of one or more of the various layers of the pull-on diaper.) More preferably, each of the seam panels 66 is an extension of the corresponding front and back side panels 46 and 48, or at least one of the component elements used therein, or any other combination of the elements. Preferably, each of the tear open tabs 31 is an extension of the corresponding seam panels 66 or at least one of their component elements used therein, or any other combination of the elements. In preferred embodiments, the front and side panels 46 and 48 are continuous members which continuously extend from the chassis 41. More preferably, at least one, preferably both of the front and back side panels 46 and 48, comprises a continuous sheet or film material 42 which is a part of the chassis 41 and continuously extends from the chassis 41. In alternative embodiments, the front and side panels 46 and 48 are discrete members (not shown in the figures) which are attached to the side edges of the chassis 41.

In a preferred embodiment, the front and back side panels 46 and 48 are elastically extensible in at least the lateral direction. As used herein, the term "elastically extensible" means a segment or portion that will elongate in at least one direction (preferably the lateral direction for the side panel) when tensional forces (typically lateral tensional forces for the side panel) are applied, and will return to about its previous size and configuration when the tensional forces are removed. More preferably, the front and back side panels 46 and 48 are elastically extensible both in the lateral and longitudinal directions.

The pull-on diaper 20 further comprises seams 32 each joining the corresponding seam panels 66 in an overlap manner to make an overlapped seam structure, thereby forming two leg openings 34 and a waist opening 36. The bonding of the seams 32 of the present invention can be performed by any suitable means known in the art appropriate for the specific materials employed in the seam panels. Thus, sonic sealing, heat sealing, pressure bonding, adhesive or cohesive bonding, sewing, autogeneous bonding, and the like may be appropriate techniques. Preferably, the seam panels 66 are joined by a pattern of heat/pressure or ultrasonic welds.

A continuous belt 38 is formed about the waist opening 36. The continuous belt 38 acts to dynamically create fitment forces and to distribute the forces dynamically generated during wear. The pull-on diaper 20 thus preferably comprises a chassis layer 40; a first belt layer 42; and a second belt layer 44. Preferably, an elastic waist feature 50 is provided in both the front region 26 and the back region 28. The pull-on diaper 20 additionally comprises elastic leg features 52. More preferably, apertures or vents (not shown) are provided in at least the side panels 46 and 48 of the pull-on diaper 20 to provide breathability and ventilation. Because the first belt layer 42 and the second belt layer 44 are preferably nonwoven webs having the appearance of cloth and the chassis layer 40 is preferably a plastic film, the pull-on diaper 20 has a unique aesthetic feature in that it is perceived by caregivers and wearers to have a garment-like comfort and feel in the waist regions while having a perceived containment benefit in the crotch region.

The continuous belt 38 is elastically extensible in the side panels 46 and 48 to provide a more comfortable and contouring fit by initially conformably fitting the pull-on diaper 20 to the wearer and sustaining this fit throughout the time of wear well past when it has been loaded with exudates by distributing forces along both the waist and legs since the sides of the pull-on diaper can expand and contract. The continuous belt 38 may be formed from a number of different materials and layers as defined below.

Fig. 2 shows a simplified plan view of the pull-on diaper 20 of Fig. 1 in its flat-out, uncontracted state depicting the various panels and their positioning with respect to each other. The pull-on diaper 20 has the crotch region 30 comprising a main panel 56 and a pair of leg flap panels 58; the front region 26 comprising a central panel comprising a waistband panel 60 and a medial panel 62, the front side panels 46, and the seam panels 66; and the back region 28 comprising a central panel comprising a waistband panel 60' and a medial panel 62', the back side panels 48, the seam panels 66', and the tear open tabs 31. The absorbent core 84 (not shown in Fig. 2) is generally positioned within the main panel 56, since exudates are typically discharged in this region, although the absorbent core 84 will typically extend into the medial panels 62 and 62' of the belt.

In the embodiment shown in Fig. 2, a leg flap panel 58 extends generally laterally outwardly from and along each side edge 68 of the main panel 56. Each leg flap panel 58 generally forms at least a portion of the elastic leg feature 52 (shown in Fig. 3). The continuous belt 38 (the front region 26 and the back region 28) extends generally longitudinally outwardly from and along each lateral edge 69 of the crotch region 30 (the main panel 56 and the leg flap panel 58). In the front region 26, the medial panel 62 extends generally longitudinally outwardly from and along the lateral edge 69 of the crotch region 30. The waistband panel 60 extends generally longitudinally outwardly from and along the medial panel 62. The side panels 46 each extend generally laterally outwardly from and along the central panel (the panels 60 and 62). The seam panels 66 each extend generally laterally outwardly from and along the respective side panel 46. In the back region 28, the medial panel 62' extends generally longitudinally outwardly from and along the other lateral edge 69 of the crotch region 30. The waistband panel 60' extends generally longitudinally outwardly from and along the medial panel 62'. The side panels 48 each extend generally laterally outwardly from and along the central panel (the panels 60' and 62'). The seam panels 66' each extend generally laterally outwardly from and along the respective side panel 48. The tear open tabs 31 each extend generally laterally outwardly from and along the respective seam panels 66'. Disposal means 33 are provided on the respective tear open tabs 31. The front region 26, in addition to its panels, also has an end edge 70, leg edges 71, and side edges 72. The back region 28, in addition to its panels, also has an end edge 70', leg edges 71', and side edges 72'. The crotch region 30 has leg edges 74.

The waistband panel 60 is preferably elastically extensible, more preferably elastically contractible or gathered, to better fit the pull-on diaper 20 in the central part of the waist opening 36. The medial panel 62 is not gathered in order to maintain the integrity of the absorbent core 84 during use. Although the medial panels 62 and 62' may be elastically extensible (but not gathered), they are preferably not extensible.

Fig. 3 is a partially cut-away plan view of the pull-on diaper 20 of Fig. 1 in its flat-out, uncontracted state (i.e., with elastic induced contraction pulled out except in the side panels 46 and 48 are left in their relaxed condition) with the backsheet 22 facing the viewer, prior to the front region 26 and the back region 28 being joined together by the seams 32. The backsheet 22 of the pull-on diaper 20 comprises that portion which is positioned away from the wearer's body during use. In the embodiment shown, the backsheet 22 of the pull-on diaper 20 comprises the first belt layer 42 in the front region 26, the second belt layer 44 in the back region 28, and the chassis layer 40 in the crotch region 30. (The inner surface 24 of the diaper is opposed to the backsheet 22 and comprises that portion of the diaper which is positioned adjacent to the wearer's body during use.)

In the embodiment shown in Fig. 3, the chassis layer 40 preferably comprises a continuous sheet or web which defines the front region 26, the back region 28, and the crotch region 30. Thus, the chassis layer 40 is the primary stratum or layer of the pull-on diaper. (As used herein, the term "layer" does not necessarily limit the element to a single strata of material in that a layer may actually comprise laminates or combinations of sheets or webs of the requisite type of materials.) The chassis layer 40 has an inner surface 76 (not shown in Fig. 3) and an outer surface 77. The inner surface 76 and outer surface 77 of the chassis layer 40 correspond in their orientation with the inner surface 24 and the backsheet 22 of the pull-on diaper 20. Since the chassis layer 40 preferably defines the front region 26, the back region 28, and the crotch region 30, the chassis layer 40 also has corresponding regions and panels as previously defined. (For simplicity, these regions and panels are denoted in the drawings by the same reference numerals as the corresponding pull-on diaper regions and panels as shown in Fig. 2.) The first belt layer 42 is positioned on the outer surface 77 of the chassis layer 40 in the front region 26 and extends continuously laterally across the front region 26 from one side edge 72 to the other side edge 72 and longitudinally from the end edge 70 to at least the leg edges 71. The first belt layer 42 is preferably joined to the chassis layer 40. The second belt layer 44 is positioned on the outer surface 77 of the chassis layer 40 in the back region 28 and extends continuously laterally across the back region 28 from one side edge 72' to the other side edge 72' and from the end edge 70' to at least the leg edges 71'. The second belt layer 44 is preferably joined to the chassis layer 40. Thus, each belt layer in combination with the chassis layer 40 forms a continuous belt 38 (as shown in Fig. 1) about the waist of the wearer. As will be detailed below, this belt has various elastic extension properties in various zones to enhance the fit and containment of the pull-on diaper 20.

Elastically extensible stretch laminates are formed in the respective side panels 46 and 48 of both the front region 26 and the back region 28. Each front side panel 46 at least comprises the portion of the first belt layer 42 in the side panel and an elastic panel member 78 joined thereto, and, in this particular embodiment, the portion of the chassis layer 40 forming the side panel. Preferably, the elastic panel member 78 is positioned between the chassis layer 40 and the first belt layer 42, and more preferably extends longitudinally from the end edge 70, most preferably to the leg edge 71. Each rear side panel 48 at least comprises the portion of the second belt layer 44 in each side panel and an elastic panel member 78' joined thereto, and, in this particular embodiment, the portion of the chassis layer 40 forming the side panel. Preferably, the elastic panel member 78' is positioned between the chassis layer 40 and the second belt layer 44, and more preferably extends longitudinally from the end edge 70', most preferably to the leg edge 71'. In the pull-on diaper embodiment shown in Fig. 3, each side panel preferably further comprises a portion of the topsheet 80 (the barrier layer) in the side panel. Each side panel is mechanically stretched or drawn (designated by the strain lines) to allow the side panel to be elastically extensible in at least the lateral direction. (The lateral direction (x direction or width) is defined as the direction parallel to the lateral centerline of the pull-on diaper; the longitudinal direction (y direction or length) is defined as the direction parallel to the longitudinal centerline; and the axial direction (z direction or thickness) is defined as the direction extending through the thickness of the pull-on diaper.)

An elastic waist feature 50 is provided in the waistband panel 60 of the front region 26 or the waistband panel 60' of the back region 28, or preferably both the front region 26 and the back region 28. The elastic waist feature 50 provides an elastically extensible member, preferably a gathered elastically contractible member, to dynamically fit and conform to the waist of the wearer in the central panels. In the embodiment shown, the elastic waist feature 50 preferably comprises a unitary waistcap/waistband 82 operatively joined in the waistband panel in an elastically contractible condition, preferably to the primary layer of the topsheet 80. The unitary waistcap/waistband 82 acts as a barrier to the leakage of exudates out of the waist opening of the pull-on diaper 20 as well as a contractible waistband to provide fit of the pull-on diaper 20 about the waist of the wearer. In the most preferred embodiments, the unitary waistcap/waistband is also breathable to allow venting of water vapor out of the pull-on diaper adjacent the waist opening.

In the embodiment shown in Fig. 3, the pull-on diaper 20 is also provided with elastic leg features 52 to improve fit at the legs in the crotch region 30. The pull-on diaper 20 additionally comprises a topsheet 80 and an absorbent core 84 (not shown) positioned between the topsheet 80 and the chassis layer 40 to provide an absorbent assembly that cooperates with the continuous belt to contain discharged exudates.

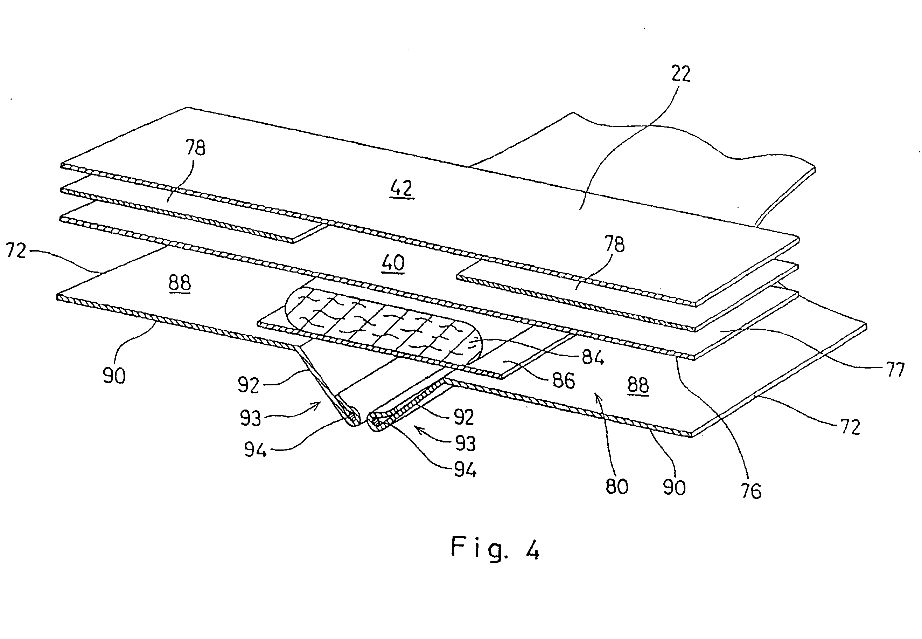

Fig. 4 is a cross-sectional perspective view of the pull-on diaper 20 taken along the line 4-4 of Fig. 3 in the front region 26. The chassis layer 40 is shown to form the primary strata or layer of the pull-on diaper 20 and has an inner. surface 76 and an outer surface 77. The first belt layer 42 is positioned on the outer surface 77 of the chassis layer 40 to form the backsheet 22 of the pull-on diaper 20 in the front region 26. The elastic panel members 78 are preferably positioned between the first belt layer 42 and the chassis layer 40. The topsheet 80 is positioned on and joined to the inner surface 76 of the chassis layer 40. The topsheet 80 preferably comprises a liquid pervious primary layer 86 and two barrier layers 88. The barrier layers 88 extend laterally outwardly from the primary layer 86 to the side edges 72. Each barrier layer 88 comprises a flap portion 90 and a stand-up portion 92. The stand-up portion 92 is not attached to the primary layer 86 to allow the gathering forces of the elastic spacing member(s) 94 to cause the stand-up portion 92 to stand up away from the surface of the primary layer 86 to form a barrier cuff 93 which is a barrier or wall in use. The flap portion 90 extends laterally outwardly from the stand-up portion 92 (the proximal edge) to the side edge 72. The absorbent core 84 is preferably positioned between the primary layer 86 and the chassis layer 40. The construction of the back region 28 is preferably identical to the construction of the front region 26.

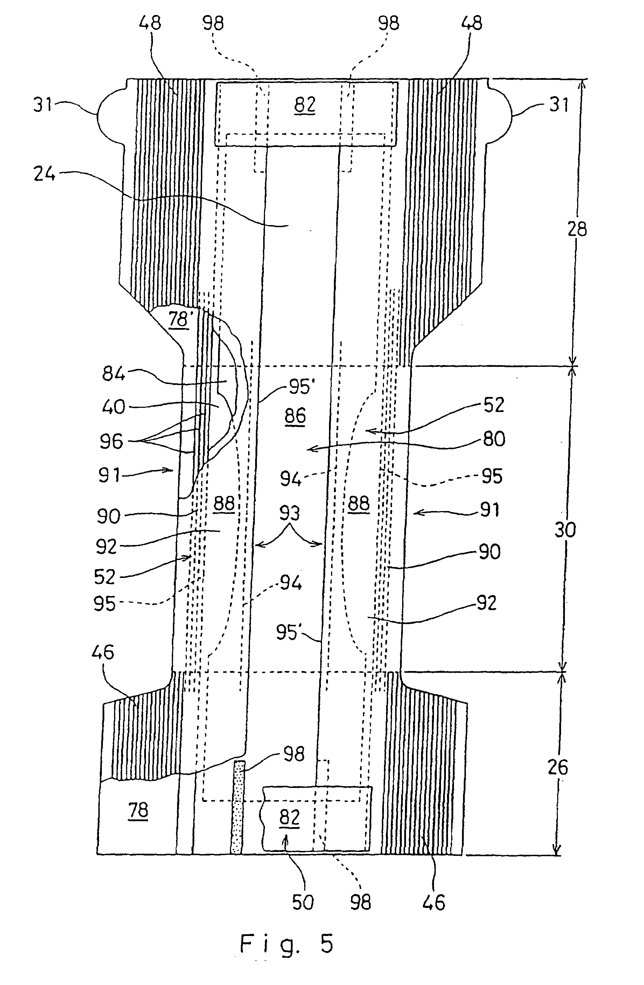

Fig. 5 is a partially cut-away plan view of the pull-on diaper 20 shown in Fig. 1 in its flat-out, uncontracted state (i.e., with elastic induced contraction pulled out except in the side panels wherein the side panels are left in their relaxed condition) with the inner surface 24 of the pull-on diaper 20 facing the viewer, prior to the front region 26 and the back region 28 being joined together by the seams. In order to provide the necessary absorbency to contain body exudates, the pull-on diaper 20 comprises a liquid pervious topsheet 80 and an absorbent core 84 positioned between the topsheet 80 and the chassis layer 40. In the embodiment shown in Fig. 5, the topsheet 80 preferably comprises three distinct layers joined together. A liquid pervious primary layer 86 is positioned over the absorbent core 84 to rapidly absorb liquids into the product. Barrier layers 88 are joined to the primary layer 86 and are preferably drawable, more preferably hydrophobic, to allow the side panels to be mechanically stretched without ripping or tearing while providing barrier cuffs 93 along the sides of the pull-on diaper 20. The barrier layers 88 have a flap portion 90 and a channel or stand-up portion 92 to provide the various components of the elastic leg features 52. The elastic leg features 52 preferably comprise a gasketing cuff 91 and a barrier cuff 93. The gasketing cuff 91 is preferably formed by one or more elastic leg members 96 operatively joined to the chassis layer 40, the barrier layer 88, or both, preferably between the chassis layer 40 and the flap portion 90 of the barrier layer 88 in the leg flap panel of the crotch region 30. The barrier cuff 93 is preferably formed by a flap (the stand-up portion 92 of the barrier layer 88), closing means 98 for securing the longitudinal ends of the stand-up portion 92 to the primary layer 86, and an elastic spacing member 94 operatively joined to the stand-up portion 92.

The elastic extensibility in the side panels 46 and 48 may be provided by a number of different materials and configurations. Various components of the belt (e.g., the belt layers or the chassis layer) may comprise conventional elastic materials or the side panels of the belt may be constructed from a number of different elastic laminate structures. For example, the side panels of the belt can comprise an elastic material operatively joined to one or more inelastic components (belt layer or chassis layer or both) in an elastically contractible condition such as is described in U.S. Patent 3,860,003 entitled "Contractable Side Portions For A Disposable Diaper" issued to Buell on January 14, 1975. Alternatively, the side panels can comprise a structural elastic-like film (SELF) web such as described in WO 95/03765, "Web Materials Exhibiting Elastic-like Behavior", The Procter & Gamble Company, published February 9, 1995. While the side panels of the belt may be constructed from a number of different extensible or elastic materials as are known in the art, one or more, and preferably each, of the side panels of the belt are constructed of a stretch laminate.

In a preferred embodiment of the present invention, an elastic panel member 78 is operatively joined in the side panels 46 and 48, preferably between the chassis layer 40 and the belt layers 42 and 44, to allow the side panels 46 and 48 to be elastically extensible in at least the lateral direction. Elastomeric materials which have been found to be especially suitable for use as the elastic panel members (especially for zero strain stretch laminates) are styrenic block copolymer based elastic films, preferably with a thickness of 0.05 mm - 0.064 mm (0.002 in - 0.0025 in), such as are made by Clopay Corporation of Cincinnati, Ohio under the designation PA18-2870; or Exxon 500 series elastic films from Exxon Chemical of Baytown, Texas. Other suitable elastomeric materials for use as the elastic panel members include "live" synthetic or natural rubber, other synthetic or natural rubber foams, elastomeric films (including heat shrinkable elastomeric films), elastomeric woven or nonwoven webs, scrims, elastomeric composites, or the like. More preferably, the elastic panel member 78 is an elastomeric scrim material. Such elastomeric scrim material is provided with a plurality of first strands that intersect a plurality of second strands, forming a net-like open structure having a plurality of apertures. Each aperture is defined by at least two adjacent first strands and at least two adjacent second strands, so that the apertures are substantially rectangular in shape. A preferred elastomeric scrim is manufactured by the Conwed Plastics Company under the designation TN2514.

In a more preferred embodiment, the elastic panel members are operatively joined in the side panel by securing them to the chassis layer 40, the belt layers 42 and 44, or both while in a substantially untensioned (zero strain) condition. At least a portion of the resultant composite stretch laminate containing the elastic panel member is then subjected to mechanical stretching sufficient to permanently elongate the non-elastic components (the chassis layer, the barrier layer, and the belt layer) of the stretch laminate. The composite stretch laminate is then allowed to return to its substantially untensioned condition. The side panel is thus formed into a "zero strain" stretch laminate. (Alternatively, the elastic panel member could be operatively joined in a tensioned condition and then subjected to mechanical stretching; although this is not as preferred as a "zero strain" stretch laminate.) As used herein, the term "zero strain" stretch laminate refers to a laminate comprised of at least two plies of material which are secured to one another along at least a portion of their coextensive surfaces while in a substantially untensioned ("zero strain") condition; one of the plies comprising a material which is stretchable and elastomeric (i.e., will return substantially to its untensioned dimensions after an applied tensile force has been released) and a second ply which is elongatable (but not necessarily elastomeric) so that upon stretching the second ply will be, at least to a degree, permanently elongated so that upon release of the applied tensile forces, it will not fully return to its original undeformed configuration. The resulting stretch laminate is thereby rendered elastically extensible, at least up to the point of initial stretching, in the direction of initial stretching. Particularly preferred methods and apparatus used for making stretch laminates utilize meshing corrugated rolls to mechanically stretch the components. Particularly preferred apparatus and methods are disclosed in U.S. Patent No. 5,167,897 issued to Weber et al. on December 1, 1992; U.S. Patent No. 5,156,793 issued to Buell et al. on October 20, 1990; and U.S. Patent No. 5,143,679 issued to Weber et al. on September 1, 1992.

The continuous belt 38 acts to dynamically create fitment forces in the pull-on diaper 20 when positioned on the wearer, to maintain the pull-on diaper on the wearer even when loaded with body exudates thus keeping the absorbent core in close proximity to the wearer, and to distribute the forces dynamically generated during wear about the waist thereby providing supplemental support for the absorbent core without binding or bunching the absorbent core in the medial panel of the pull-on diaper. The belt is designed to be elastically extensible in certain segments and at least elastically extensible, preferably elastically contractible, in other segments about the waist opening; to be elastically extensible about a portion of the leg opening; and to not be gathered or bunched in the medial panel where the absorbent core is located. The elastic extensibility of the belt also has a "force/extension wall" beyond which the belt will not elastically extend in order to allow the pull-on diaper to be more easily applied since the diaper will not stretch excessively thereby allowing the product to slide more easily over the buttocks. This "force/extension wall" is especially important for small children who self apply the product and would be unable to completely pull and position a fully stretchable product over their buttocks. The belt also manages wearing stresses better with the belt webs encircling the absorbent core. The resultant diaper is less bulky in its fit about the waist of the wearer. The lack of gathering or contraction in the belt over the absorbent core in conjunction with the continuity of the belt across the absorbent core also provides improved fit by providing a continuous normal force based on the hoop stresses generated in the belt which tends to press the absorbent core against the body during wear. Thus, the absorbent core is maintained in a closer, more comfortable, and less gapping way than those diapers which provide elastic contraction or gathering over the area of the absorbent core or those that do not provide a belt to concentrate within the span and distribute across the span the hoop stresses about the area of the absorbent core.

The seam panels 66 and 66' are those portions designed to be seamed or joined together by the manufacturer to form the defined waist opening 36 and leg openings 34. As shown in Fig. 2, the seam panels 66 and 66' extend laterally outwardly from the respective side panels 46 and 48 to the side edge 72 or 72' and generally longitudinally extend from the end edge 70 or 70' to the leg edge 71 or 71', respectively. The seam panels are preferably an extension of the chassis layer and other elements such as the belt layers and the topsheet, or any other combination of these elements. In a preferred embodiment, each seam panel is formed by portions of the chassis layer, the belt layers, the elastic panel members, and the barrier layers of the topsheet. (In the seam panel, the stretch laminate is preferably not activated by mechanical stretching, although it may be, if desired, to provide additional extensibility in this region.)

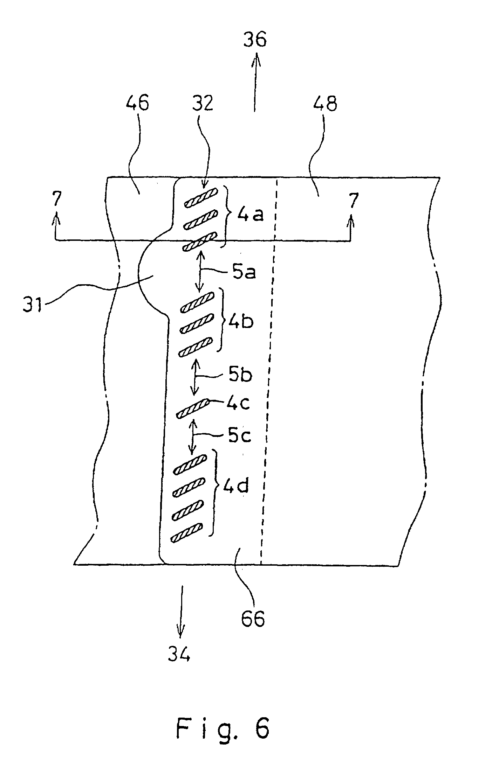

The seam 32 has a predetermined seam pattern so that the seam can be torn open easily by using the tear open tab 31. The seam 32 comprises an intermittent pattern of bonds grouped in clusters 4a, 4b, 4c and 4d as shown, for example, in Fig. 6. In this embodiment, the seam 32 comprises four clusters 4a-4d. Other suitable seams may have any number of clusters such as two, three, five, or more. Any shape of bond and/or cluster can be used as long as the seam can be torn open easily by using the tear open tab 31. Preferred shapes of bonds and/or clusters include a circle, an oval, a triangle and a rectangle.

The spacing between adjacent clusters 4a-4d creates gaps or unbonded portions 5a-5c in the seam 32. When the diaper 20 is worn, the seams 32 tend to buckle with the movement of the wearer. The buckling of the seams 32 at the spacings between adjacent clusters creates channels allowing air to move in and out of the diaper during use to ventilate the diaper. Thus, the unbonded portion of the seam 32, (i.e., the summation of all the individual spacing distances), determines the breathability of the seams, and thus, the breathability of the diaper 20. As the unbonded portion of the seam 32 increases so does the breathability of the diaper. However, as the unbonded portion of the seam 32 increases, the strength of the seam 32 decreases. Preferably, the unbonded portion of the seam 32 ranges from about 20% to about 65% of the total seam length, as measured from the leg opening 34 to the waist opening 36 along seam 32. Examples of suitable seams have an unbonded portion comprising about 30%, 35% or 40% of the total seam length. Unbonded portions below about 20% are generally not desirable as they do not allow a sufficient amount of air in to ventilate the diaper. Unbonded portions above about 65% are generally not desirable either as they allow sufficient amounts of air in to ventilate the diaper, but are not sufficiently strong to withstand the high forces and stresses placed on the diaper during wear.

Preferred seam patterns may include different seam strength patterns which provide varying bonding strengths between the seam panels 66 so that the seam can be torn open easily by using the tear open tab 31. In preferred embodiments, such control of the bonding strengths may be produced by changing certain amounts of polymeric material contained in the seam panels 66. To increase the bonding strength, for example, the amount of polymeric material contained in the elements forming the seam panels 66 may be increased by using higher basis weight nonwoven materials, thicker plastic films, or by introducing additional layers of materials to the seam panels 66. For example, additional plastic films or nonwoven webs may be joined in the seam panels 66. Alternatively, the layers forming the diaper may be extended beyond the intended area of seaming and folded back into the seam panel to introduce additional strata in the seam panels. Examples of these types of seams are discussed in the above-referenced U.S. Patent No. 5,236,430.

In preferred embodiments, the tear open tab 31 can be positioned at any place along the edge of the seam panel 66. In the embodiment shown in Fig. 6, the tear open tab 31 is positioned at an outwardly extended portion of the spacing 5a which is about equally close to both the clusters 4a and 4b.

Preferably, the position of the tear open tab 31 is biased between the leg opening 34 and the waist opening 36. In a preferred embodiment, the position of the tear open tab is closer to the leg opening 34 than the waist opening 36. In a more preferred embodiment, the position of the tear open tab 31 is closer to the waist opening 36 than the leg opening 34 as shown in Fig. 6. The biased position of the tear open tab 31 indicates the user from which portion he/she should start tearing open the diaper by the fingers.

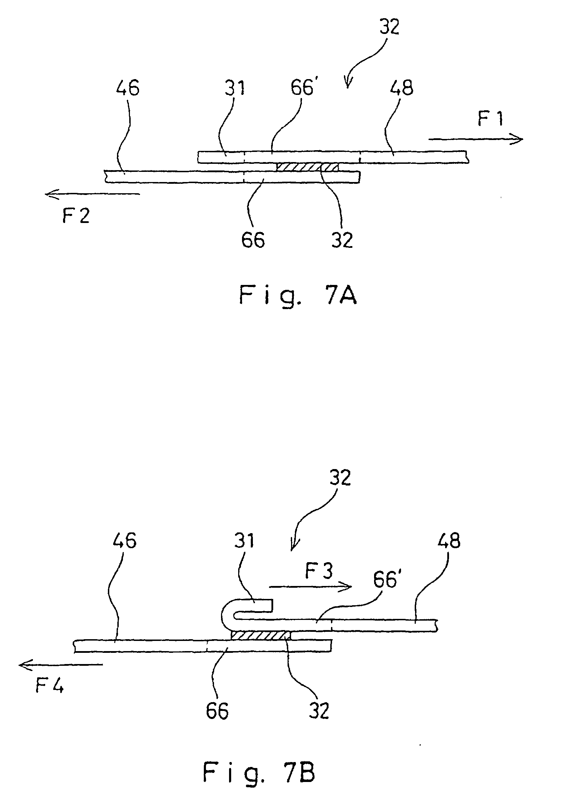

Fig. 7A shows the overlapped seam structure 32 formed by joining the seam panels 66. In this figure, one of the seam elements 32 is exaggeratedly depicted in terms of its thickness and width for the sake of explanation. The arrows F1 and F2 show the forces which are generated when the diaper 20 is worn, thus applied to the seam element 32 in the seam panels 66 and 66' through the front and back side panels 46 and 48. The overlapped seam structure provides a necessary seam strength which is required to maintain the pull-on diaper on the waist area of the wearer. Fig. 7B shows the forces F3 and F4 applied to the tear open tab 31 and the front side panel 46, respectively, when the pull-on diaper is torn open during the removal of the diaper from the wearer. As the opposite forces F3 and F4 effectively work to separate the seam panel 66 of the front side panel 46 from the seam panel 66' of the back side panel 48. The biased position of the tear open tab 31 causes a concentration of the tear open force F3 at the specific seam element 32 that is positioned closest to the leg opening 34 or the waist opening 36. Thus, the pull-on diaper 20 can be tom open from the leg opening 34 or the waist opening 36 by the application of a small amount force from the user.

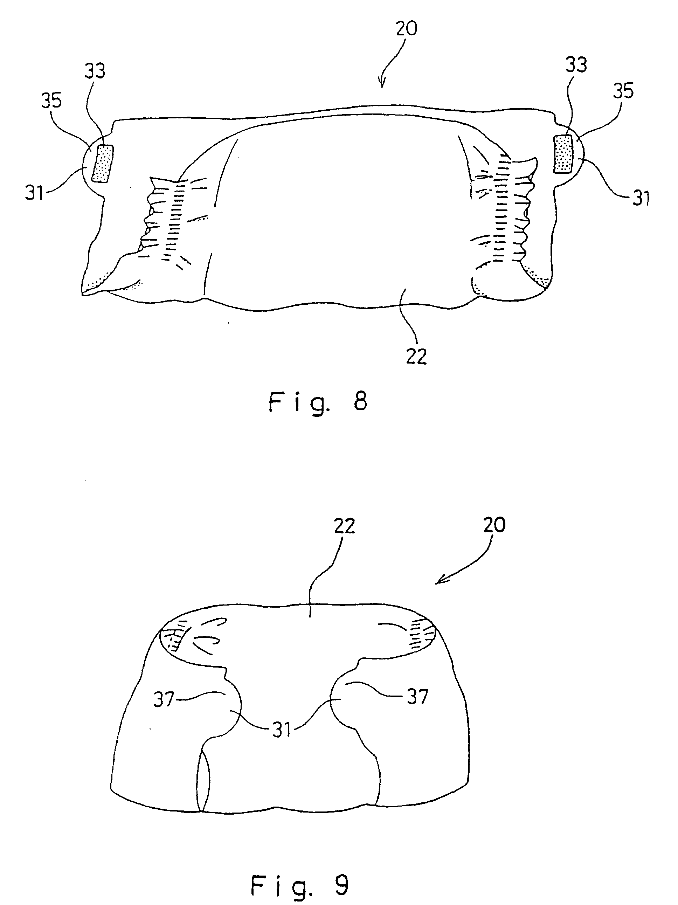

In more preferred embodiments, the pull-on diaper 20 further comprises a disposal means 33 joined to the tear open tab 31 for allowing the pull-on diaper to be secured in a configuration that provides convenient disposal. A preferred example of the disposal means 33 is shown in Fig. 8. In preferred embodiments, the tear open tab 31 has an inner surface 35 and an outer surface 37 (shown in Fig. 9). The disposal means 33 is joined to the inner surface 35 of the tear open tab 31 so that the disposal means 33 is positioned between the tear open tab 31 and the front side panels 46, and hidden therebetween as shown in Fig. 1.

The disposal means 33 may be any material which can allow the pull-on diaper 20 to be secured in a configuration that provides for convenient disposal. In preferred embodiments, the disposal means is a material which can engage with or stick to at least a part of the backsheet 22 or one of the front and back side panels 46 and 48 for disposal. In a preferred embodiment, the disposal means 33 comprises a mechanical fastening element which constitutes a mechanical fastener system with the backsheet 22 for disposal. In an alternative preferred embodiment, the disposal means 33 comprises a mechanical fastening element which constitutes a mechanical faster system with one of the front and back side panels 46b and 48 for disposal. The mechanical fastening element may comprise any of known means for achieving a closure such as buttons, snaps, hook fastening materials, or loop fastening materials.

The mechanical fastener system may comprise any mechanical fasteners known in the art. Exemplary fastening systems comprising mechanical fastening components are described in U.S. Patent No. 5,058,247 entitled "Mechanical Fastening Prong" issued to Thomas October 22, 1991; U.S. Patent No. 4,869,724 entitled "Mechanical Fastening Systems With Adhesive Tape Disposal Means For Disposal of Absorbent Articles" issued to Scripps on September 26, 1989; and U.S. Patent No. 4,846,815 entitled "Disposable Diaper Having an Improved Fastening Device" issued to Scripps on July 11, 1989.

In one preferred embodiment, the mechanical faster system comprises hook and loop type fasteners. As used herein, the term "hook and loop type fasteners" refers to fastening means comprising a "hook" fastening material and a complementary loop fastening material. The term "hook" is used to designate a material having engaging elements. Thus, the hook fastening material may also be referred to as a male fastener. It should also be understood that the use of the term "hook" should be non-limiting in the sense that the engaging elements may comprise any shapes as are known in the art so long as they are adapted to engage a complementary landing component. Thus, the hook fastening material may be manufactured from a wide range of materials. Further, the engaging elements may have any shape such as hooks, "T's", "mushrooms" or any other shape as are well known in the art. Suitable materials include nylon, polyester, polypropylene, or any combination of these materials. An example of preferred hook fastening material is available from Sumitomo 3M, under the trade designation XPH-5089. Yet another preferred hook fastening material is described in U.S. Patent No. 5,058,247 entitled "Mechanical Fastening Prong" issued to Thomas October 22, 1991.

In another preferred embodiment, the disposal means comprises a hook fastening material whereas the backsheet 22 or one of the front and back panels 46 and 48 comprises a loop fastening material at least at an expected landing portion. More preferably, the hook fastening material comprises a base and a plurality of engaging elements extending from the base. The hook fastening material is intended to engage with fibrous elements of a loop fastening material which is formed in or on the backsheet 22 or one of the front and back panels 46 and 48. The base may be joined to the tear open tab 31 by any suitable means. In preferred embodiments, they are directly joined with the inner surface of the tear open tab 31.

In other preferred embodiments, the backsheet 22 or the front and back panels 46 and 48 comprises a landing component (not shown) at least at the expected landing portion of the disposal means 33 for disposal. The landing component can engage with or stick to the disposal means 33. The landing component may be manufactured from a wide range of materials and configurations capable of securely engaging the engaging component. For example, the landing component may comprise identical complementary elements or distinct complementary elements. As used herein, the term "identical complementary elements" is used to define mechanical fastening systems wherein the engaging elements of the engaging component and the landing component comprise the same configuration or structure. The term "distinct complementary elements" is used herein to designate a system wherein the engaging component is different from the landing component but is engageable therewith.

In one preferred embodiment, the landing component comprises a plurality of fiber elements, such as a loop fastening material, that engage with the engaging elements of the disposal means 33. The loop fastening material may be manufactured from a wide range of materials to provide fiber elements, preferably loops. Suitable materials include woven materials, nonwovens, nylons, polyesters, polypropylenes, or any other known loop fastening materials or combination of these materials. A preferred loop fastening material comprises a number of shaped engaging elements projecting from a woven backing. An inexpensive loop fastening material and a method of making the same is described in U.S. Patent No. 5,032,122, entitled "Loop Fastening Material For Fastening Device and Method of Making Same" issued to Noel et al., July 16, 1991. Another suitable landing component material is described in U.S. Patent No. 5,326,612 entitled "Nonwoven Female Component for Refastenable Fastening Device and Method of Making the Same" issued to David J. K. Goulait on July 5, 1994.

In another preferred embodiment, the landing component has a base and a plurality of engaging elements extending from the base. The base is joined to the backsheet 22 or the front and back panels 46 and 48 at the expected landing portion. The landing component may be joined with the backsheet 22 or the front and back panels 46 and 48 by any means known in the art, including but not limited to adhesives, cohesives, heat, pressure, ultrasound, or any combination thereof. Alternatively, the landing component may be formed in a part of the backsheet 22 and the front and back panels 46 and 48.

In preferred embodiments, the backsheet 22 comprises a plastic film and a nonwoven web joined to the outer-facing surface of the plastic film so that the nonwoven web covers at least a portion, preferably more than 70%, more preferably substantially all of the outer-facing surface of the plastic film. The nonwoven web has at least a portion which forms the landing component. Preferably the plastic film is moisture permeable. The nonwoven web may be joined to the plastic film by any suitable attachment means known in the art. For example, the nonwoven web may be secured to the plastic film by a uniform continuous layer of adhesive, a patterned layer of adhesive, or an array of separate lines, spirals, or spots of adhesive. Suitable nonwoven web include a spunbond non woven web of polypropylene obtainable from Fiberweb Co., Ltd., SC, USA as 13561 DAPP. Another suitable spunbond non woven web of polypropylene is obtainable from Veratec Co., Ltd., MA, USA as L4819.

In an alternative embodiment, the disposal means comprises an adhesive attachment means which can stick to or adhere to the backsheet 22 or the front and back panels 46 and 48 for disposal. In a more preferred embodiment, the adhesive attachment means comprises an adhesive positioned on the inner surface of the tear open tab 31, and a release liner positioned on the adhesive so that the adhesive will not be exposed until after the disposable pull-on diaper has been soiled. The adhesive attachment means may comprise any adhesive or glue used in the art. Preferably, pressure-sensitive adhesives are used. The release liner serves to keep the adhesive attachment means from drying out and sticking to extraneous surfaces prior to use. Any release liner commonly used for such purposes can be used.

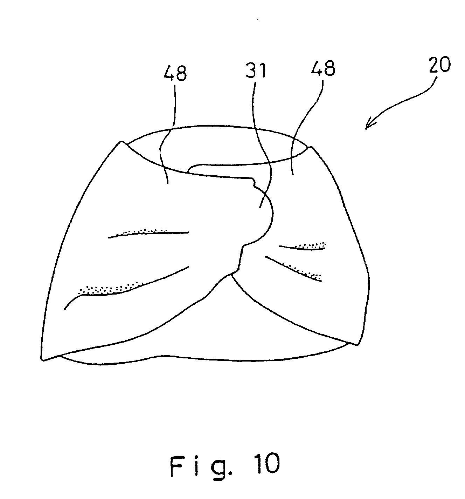

After the pull-on diaper 20 is used or soiled, the soiled diaper 20 is torn open along the seams 32 by gripping the tear open tab 31 and the front side panel 46 to remove the soiled diaper 20 from the wearer. The removed diaper 20 is then folded on rolled up into a configuration for disposal to contain the contents within the soiled diaper as shown, for example, in Fig. 8. To secure the folded or rolled up diaper 20 with the disposal means 33, any configuration can be chosen. For example, the tear open tabs 31 are bonded to the backsheet 22 through the disposal means 33 (not shown) to secure the disposal configuration as shown in Fig. 9. Alternatively, one of the tear open tabs 31 of one side is bonded to the back side panel 48 of the other side through the disposal means 33 to secure the disposal configuration shown in Fig. 10. In these examples (shown in Figs. 9 and 10), the disposal means 33 may be either a mechanical fastening element or an adhesive which is provided on the tear open tab 2 and is designed to engage with or stick to the backsheet 22 or the back side panel 48.

The absorbent core 84 is preferably positioned adjacent the inner surface 76 of the chassis layer 40 and is preferably joined thereto by attachment means (not shown) such as those well known in the art. For example, the chassis layer may be secured to the absorbent core by a uniform continuous layer of adhesive, a patterned layer of adhesive, or an array of separate lines, spirals, or spots of adhesive. The attachment means preferably comprises an open pattern network of filaments of adhesive as is disclosed in U.S. Patent No. 4,573,986 entitled "Disposable Waste-Containment Garment", which issued to Minetola and Tucker on March 4, 1986. An exemplary attachment means of an open pattern network of filaments comprises several lines of adhesive filaments swirled into a spiral pattern such as is illustrated by the apparatus and methods shown in U.S. Patent No. 3,911,173 issued to Sprague, Jr. on October 7, 1975; U.S. Patent No. 4,785,996 issued to Ziecker, et al. on November 22, 1978; and U.S. Patent No. 4,842,666 issued to Werenicz on June 27, 1989. Alternatively, the attachment means may comprise heat bonds, pressure bonds, ultrasonic bonds, dynamic mechanical bonds, or any other suitable attachment means or combinations of these attachment means as are known in the art.

The absorbent core 84 may be any absorbent means which is generally compressible, conformable, non-irritating to the wearer's skin, and capable of absorbing and retaining liquids such as urine and other certain body exudates. The absorbent core may be manufactured in a variety of sizes and shapes (e.g., rectangular, hour-glass, "T"-shaped, asymmetric, etc.) and from a wide variety of liquid absorbent materials commonly used in disposable diapers and other absorbent articles such as comminuted wood pulp which is generally referred to as airfelt. Examples of other suitable absorbent materials include creped cellulose wadding, meltblown polymers including coform, crosslinked cellulosic fibers, tissue including tissue wraps, absorbent foams, absorbent sponges, superabsorbent polymers, absorbent gelling materials, or any equivalent materials or combinations of materials. The configuration and construction of the absorbent core may also be varied (e.g., the absorbent core may have varying caliper zones, hydrophilic gradients, superabsorbent gradients, or lower average density and lower average basis weight acquisition zones; or may comprise one or more layers or structures). The total absorbent capacity of the absorbent core should, however, be compatible with the design loading and the intended use of the pull-on diaper. Further, the size and absorbent capacity of the absorbent core may be varied to accommodate wearers ranging from infants through adults.

A preferred embodiment of the absorbent core has an asymmetric, modified hourglass shape and has a body surface toward the body of the wearer (inner surface) and a diaper surface opposite the body surface. An exemplary absorbent structure for use as the absorbent core of the present invention that has been widely accepted is described in U.S. Patent No. 5,360,420 entitled "Absorbent Structures Containing Stiffened Fibers and Superabsorbent Material" issued to Cook, Lash, Moore, & Young on November 1, 1994. Preferably, the absorbent core will comprise an acquisition/distribution layer of chemically stiffened cellulosic fibers and a storage layer positioned beneath the acquisition/distribution layer comprising a mixture of wood pulp fibers and superabsorbent material such as are disclosed in U.S. Patent No. 4,610,478 entitled "High-Density Absorbent Structures" issued to Weisman and Goldman on September 9, 1986.

The topsheet 80 is positioned adjacent the body surface of the absorbent core 84 and is preferably joined to the absorbent core 84 and the chassis layer 40 by attachment means (not shown) such as those well known in the art. In a preferred embodiment, the topsheet and the chassis layer are indirectly joined together by directly joining them to the absorbent core or the elastic panel members or other elements of the pull-on diaper.

The topsheet 80 preferably comprises a three member structure such as disclosed in U.S. Patent No. 4,795,454, entitled "Absorbent Article Having Leakage-Resistant Dual Cuffs" issued to Dragoo on January 3, 1989. As shown in Fig. 5, the topsheet 80 comprises a primary layer 86 and barrier layers 88 joined to and extending laterally outwardly from the primary layer 86. The primary layer 86 is a liquid pervious material allowing liquids to rapidly penetrate through its thickness and be absorbed by the absorbent core. The two barrier layers 88 are preferably hydrophobic to prevent leakage out the sides of the diaper and are more preferably drawable to strengthen the stretch laminates.

## Claims

1. A disposable pull-on garment (20) comprising an outer surface, an inner surface, a front portion (11), a rear portion (12), a crotch portion (13), each of the front portion (11) and the rear portion (12) having side panels with side edges (14), and overlapping side seams (15, 32) which join together the side panels of the front portion (11) and the rear portion (12) to form leg openings (16) and a waist area, wherein
at least one side flap (17) is disposed outboard of each of the side edges (14) of the side panels forming the outermost portion of the overlapping side seams (15), and wherein said side flap (17) is a tear tab (31),
**characterized in that** said side seam (15,32) comprises an intermittant pattern of bonds grouped in clusters (4a-4d), wherein the spacing between adjacent clusters (4a-4d) creates unbonded portions (5a-5c).

2. The disposable pull-on garment (20) according to Claim 1 wherein the side flap (17) is an integral part of the side edges (14).

3. The disposable pull-on garment (20) according to any of the previous Claims wherein the side flap (17) has a curved shape for easy handling.

4. The disposable pull-on garment (20) according to any of the previous Claims wherein the side flap (17) is disposed along the side edge of the side panel corresponding to either the waist area, the leg area or area between the waist area and the leg openings.

## Patentansprüche

1. Wegwerfbares Anziehkleidungsstück (20) mit einer Außenfläche, einer Innenfläche, einem vorderen Abschnitt (11), einem hinteren Abschnitt (12), einem Schrittbereich (13), wobei der vordere Abschnitt (11) und der hintere Abschnitt (12) jeweils Seitenfelder mit Seitenrändern (14) aufweisen, und überlappenden Seitennähten (15, 32), die die Seitenfelder des vorderen Abschnittes (11) und des hinteren Abschnittes (12) miteinander verbinden, um Beinöffnungen (16) und einen Taillenbereich zu bilden, wobei mindestens eine Seitenlasche (17) außerhalb von jedem der Seitenränder (14) der Seitenfelder angeordnet ist, die den äußersten Abschnitt der überlappenden Seitennähte (15) bilden, und wobei die Seitenlasche (17) ein Aufreiß-Streifen (31) ist, **dadurch gekennzeichnet, dass** die Seitennaht (15, 32) ein intermittierendes Muster von in Gruppen (4a-4d) angeordneten Bindungen aufweist, wobei der Abstand zwischen benachbarten Gruppen (4a-4d) ungebundene Abschnitte (5a-5c) erzeugt.

2. Wegwerfbares Anziehkleidungsstück (20) nach Anspruch 1, wobei die Seitenlasche (17) ein integraler Abschnitt der Seitenränder (14) ist.

3. Wegwerfbares Anziehkleidungsstück (20) nach einem der vorherigen Ansprüche, wobei die Seitenlasche (17) eine gekrümmte Form zum einfachen Handhaben aufweist.

4. Wegwerfbares Anziehkleidungsstück (20) nach einem der vorherigen Ansprüche, wobei die Seitenlasche (17) entlang des Seitenrandes des Seitenfeldes vorgesehen ist, der entweder dem Taillenbereich, dem Beinbereich oder einem Bereich zwischen dem Taillenbereich und den Beinöffnungen entspricht.

## Revendications

1. Vêtement à enfiler jetable (20) comprenant une surface extérieure, une surface intérieure, une partie avant (11), une partie arrière (12), une partie d'entrejambe (13), la partie avant (11) et la partie arrière (12) comportant des panneaux latéraux présentant des bords latéraux (14) et des coutures latérales (15, 32) se chevauchant qui réunissent ensemble les panneaux latéraux de la partie avant (11) et de la partie arrière (12) afin de former des ouvertures de jambe (16) et une zone de ceinture, dans lequel
au moins un rabat latéral ( 17) est placé à l'extérieur de chacun des bords latéraux (14) des panneaux latéraux formant la partie la plus extérieure des coutures latérales (15) se chevauchant, et dans lequel ledit rabat latéral (17) est une patte d'ouverture par déchirage (31), **caractérisé en ce que** ladite couture latérale (15, 32) comprend un motif intermittent de liaisons formant plusieurs groupes (4a à 4d), dans lequel l'espacement entre les groupes adjacents (4a à 4d) engendre des parties non liées (5a à 5c).

2. Vêtement à enfiler jetable (20) selon la revendication 1, dans lequel le rabat latéral (17) est une partie solidaire des bords latéraux (14).

3. Vêtement à enfiler jetable (20) selon l'une quelconque des revendications précédentes, dans lequel le rabat latéral (17) a une forme incurvée en vue d'une manipulation facile.

4. Vêtement à enfiler jetable (20) selon l'une quelconque des revendications précédentes, dans lequel le rabat latéral (17) est placé le long du bord latéral du panneau latéral correspondant, soit à la zone de ceinture, soit à la zone de jambe, soit à la zone située entre la zone de ceinture et les ouvertures de jambe.
